# EUROPEAN PATENT APPLICATION

(11) **EP 3 800 274 A1**
(43) Date of publication of application: **07.04.2021**
(21) Application number: 20199625.3
(22) Date of filing: 01.10.2020
(51) Int. Cl.: C12R 1/01, C12N 1/00, C12P 7/62, C12P 7/64, C12P 11/00, C12P 19/04, C12M 1/00, C12M 1/12

(54) **METHOD FOR PRODUCING BIOMASS OR DERIVATIVES THEREOF**

(30) Priority: 01.10.2019 BE 201905647
(71) Applicant: Avecom NV, 9032 Wondelgem (BE)
(72) Inventor: Verstraete, Willy, 9032 Wondelgem (BE)
(74) Representative: Brantsandpatents bvba

(57) **Abstract**

The current invention provides for a method for producing biomass or derivatives thereof, comprising converting a sulfur compound to hydrogen sulfide by sulfate reducing microorganisms and subsequently converting said hydrogen sulfide into biomass by means of sulfide oxidizing bacteria (SOB), wherein said conversions are mediated by electron transfer of one or more energy rich gases. The current invention also provides a bioreactor system for producing biomass or derivatives thereof.

## Description

### FIELD OF THE INVENTION

The present invention relates to the production of biomass or derivatives thereof, by means of the sulfur cycle, and sulfate reducing microorganisms and sulfide oxidizing bacteria.

### BACKGROUND

In nature, particularly in the oceans, about 50% of all dark CO₂ fixation, i.e. CO₂ fixed not by the algae etc., is due to the sulfur cycle (Volkov and Rozanov 1983). Thus, the significance of the sulfur cycle in CO₂ fixation in the biosphere is enormous. The microbial mediated hydrogen cycling in deep sea hydrothermal vents is also well documented (Adam and Perner 2018). The concept of using sulfur metabolizing bacteria as pacemakers for the generation of valuable industrial products is in line with many natural processes, but yet largely unexplored.

There is a lot of literature about the dissimilatory sulfate reduction process and thiosulfate and sulfate reducing bacteria (SRB). The possibility to recover resources such as biohydrogen, hydrocarbons, polyhydroxyalkanoates, magnetite and metal sulfides via this reduction process have been described (Qian et al. 2019). There are further publications about using sulfide oxidizing bacteria to produce sulfur (Jansen et al. 2001), sulfate, and even microbial protein (Sublette 1987). Sulfide-driven microbial synthesis in which sulfide serves as electron donor on the anode to generate organics at the cathode has also been described (Gong et al. 2012). In addition, a sulfur cycle-based total removal of organics in which thiosulfate is the revolving intermediate has been described (Jin Qian et al. 2019).

Gases rich in chemical energy can, alone or in combination, be used to biosynthesize a variety of products. Such energy rich gases are for example hydrogen, CO, H₂S, PH₃, CH₄, volatile organics, mixes of such gases, or gases produced by fermentation (H₂/Biogas) or by physical/chemical gasification processes e.g. syngas made from fossil or from renewable resources or wastes.

In the domain of microbial biotechnology, such energy rich gases can serve as electron donors and can give rise to the generation of added value microbial biomass resp. microbial products, by means of biochemical conversions in which these electrons are transferred to electron acceptor compounds. Examples are the fermentation of syngas to bioethanol, of hydrogen with CO₂ to biomethane, of the mix of methane/CO₂ and hydrogen (biohythane) to methanol (Patel et al. 2019), of hydrogen to long chain fatty acids, and of methane and methanol to microbial biomass. All these examples relate to processes which transfer the electrons from the energy rich gaseous electron donor compounds directly to electron accepting species. A two-step procedure is known in which hydrogen and CO₂ are first converted to acetate by *Clostridium ljundahlii* and subsequently acetate is aerobically converted to food grade *Saccharomyces cerevisiae* yeast (Molitor et al. 2019). However, in these processes the sulfate reducing bacteria are not described and not of interest.

Gases such as hydrogen, carbon monoxide, methane and the like, and various volatile organics have solubilities of a few mg chemical oxygen demand (COD) equivalent per L water. Hence, they are poorly accessible for microorganisms in aqueous environments when the latter try to convert them into cells or microbial products. This results in in the necessity to use reactors operated with intensive gas mixing (large opex - operational expense) resp. high pressure (considerable capex - capital expense).

Biomass as a living entity is a potentially valuable product. It can be a source of bio-catalysis, such as for bringing about CO₂ fixation processes, metal and mineral insolubilization or solubilization, and other alterations of materials and compounds. Biomass components of general interest are for example proteins, polyhydroxy butyrate and analogues, polyphosphates, cell wall components such as exopolysaccharides in general and sulfated polysaccharides in particular, and extracellular dissolved organic carbon compounds.

A variety of microbial metabolites and (biomass) components are produced on industrial scale. Energy rich gases, as electron donors, can be at the onset of many of these end products by clever selection of the proper microbial cell factory mechanisms, be it in a single strain or in combinations thereof. The search for organisms specially equipped to metabolize such energy rich gases, the rates of formation and yields of the overall process to generate microbial products becomes of increased industrial interest.

Accordingly, a need arises for a method for producing biomass and thereof such microbial end products, using a route which maximizes the gas conversion capabilities of a specific microbial cell factory mechanism, preferably with a limited cost of potential overall energy gain.

### SUMMARY OF THE INVENTION

The present invention and embodiments thereof serve to provide a solution to one or more of above-mentioned disadvantages. To this end, the present invention relates to a method for producing biomass or derivatives thereof, according to claim 1.

In particular the method comprises converting a sulfur compound to hydrogen sulfide by sulfate reducing microorganisms and subsequently converting said hydrogen sulfide into biomass by means of sulfide oxidizing bacteria, wherein said conversions are mediated by electron transfer of one or more energy rich gases.

Embodiments of the method are shown in any of the claims 2-14.

In a second aspect, the present invention relates to a system for producing biomass or derivatives thereof according to claim 15.

This invention deals with the clever use of the whole of the sulfur cycle, and particularly the combination of the reduction of sulfur compounds such as sulfate or thiosulfate by microorganisms in which energy rich gases such as hydrogen, carbon monoxide, methane,.. are densified to molecules which subsequently are used by sulfur oxidizing bacteria (SOB) and associated species to produce valuable resources such as biomass derivatives by using the products of the sulfate reducing microorganisms in combination with high redox electron acceptors such as oxygen or nitrate. This latter reaction step can also be performed using light.

The biomass may subsequently be used in feed (fish feed, animal feed, pet food) and/or food for human consumption and/or as slow release organic fertilizer. Biomass derivatives may have applications in feed and/or food, in agricultural, nutritional, cosmetic and/or pharmaceutical applications or as biopolymer. The invention thereby contributes to the economical optimal use of nutrient raw materials.

### FIGURE

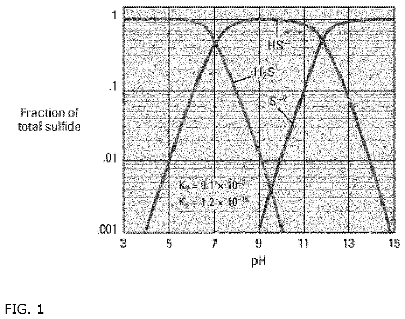

**Figure 1** shows the distribution of three sulfide species in water as function of pH.

### DEFINITIONS

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of ± 20 % or less, preferably ± 10 % or less, more preferably ± 5 % or less, even more preferably ± 1 % or less, and still more preferably ± 0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

"Sulfate-reducing microorganisms" (SRM) or "sulfate-reducing prokaryotes" (SRP) are a group composed of sulfate-reducing bacteria (SRB) and sulfate-reducing archaea (SRA), both of which can perform anaerobic respiration utilizing sulfate (SO₄²⁻) as terminal electron acceptor, reducing it to hydrogen sulfide (H₂S). Therefore, these sulfidogenic microorganisms 'breathe' sulfate rather than molecular oxygen (O₂), which is the terminal electron acceptor reduced to water (H₂O) in aerobic respiration. Most sulfate-reducing microorganisms can, aside of sulfate (SO₄²⁻ ) also reduce some other oxidized inorganic sulfur compounds, such as sulfite (SO₃²⁻ ), dithionite (S₂O₄²⁻), thiosulfate (S₂O₃²⁻), trithionate (S₃O₆²⁻), tetrathionate (S₄O₆²⁻), elemental sulfur (S₈), and polysulfides (Sₙ²⁻).

In the context of the present invention, "biomass" or "microbial biomass" refers to the mass of biological organisms, both living or dead, in a given area or ecosystem at a given time, in particular the mass of microorganisms such as archaea, algae, yeasts, fungi and/or bacteria.

"Single-cell proteins" (SCP) or "microbial proteins" refers to edible unicellular microorganisms. The biomass or protein extract from pure or mixed cultures of algae, yeasts, fungi or bacteria may be used as an ingredient or a substitute for protein-rich foods, and is suitable for human consumption or as animal feeds (fish feed, animal feed and/or pet food). It may also be suitable as slow release organic fertilizer.

"Slow release fertilizer" refers to fertilizers that release a small, steady amount of nutrients over a course of time. These can be natural, organic fertilizers that add nutrients to their environment, such as to soil, by (naturally) breaking down and/or decomposing.

"Biomass derivative", in the present context also referred to as "biomass component" or "biomass product" refers to any product or component which may be derived from biomass as defined above and products these organisms have produced and possibly secreted during or after their lifespan, the derivatives such as but not limited to proteins, polyhydroxy butyrate and analogues, polyphosphates, carbohydrates, cell wall components such as exopolysaccharides in general and sulphated polysaccharides in particular, lipids, extracellular dissolved organic carbon compounds and metabolites.

As a non-limiting examples, a biomass derivative may be an extracellular polymeric substance or a polysaccharide on the cell wall or cell membrane of the organism, a lipid inserted in said cell wall or membrane, or a protein produced and secreted by such organism. Biomass derivatives may have applications in feed and/or food, in agricultural, nutritional, cosmetic and/or pharmaceutical applications or as biopolymer.

"Densification" in the context of the present invention relates to an increase in amount of Chemical Oxygen Demand (COD) per unit volume (mL, L) of a compound.

"Chemical oxygen demand" is an indicative measure of the amount of oxygen that can be consumed by reactions in a measured solution. It is commonly expressed in mass of oxygen consumed over volume of solution which in SI units is milligrams per liter (mg/L). A COD test can be used to easily quantify the amount of organics in water.

In the context of the present invention, the term "energy rich gases" or "energy dense gases" refers to gases rich in chemical energy such as hydrogen, CO, H₂S, PH₃, CH₄, volatile organics, mixes of such gases, gases produced by fermentation such as H₂ and biogas, or by physical/chemical gasification processes such as syngas made from fossil or from renewable resources or wastes. Such energy rich gases can serve as so-called electron donors and by means of biochemical conversions in which these electrons are transferred to proper electron acceptor compounds, can give rise to the generation of added value microbial products.

### DETAILED DESCRIPTION OF THE INVENTION

The present invention concerns a method for producing biomass or derivatives thereof. Said derivatives are preferably chosen from the group of proteins, single-cell protein, polyhydroxy butyrate and analogues, polyphosphates, carbohydrates, cell wall components preferably exopolysaccharides preferably sulfated polysaccharides, lipids, extracellular dissolved organic carbon compounds and metabolites.

In a first aspect, the present invention relates to a method for producing biomass or derivatives thereof, comprising converting a sulfur compound to hydrogen sulfide by sulfate reducing microorganisms, and subsequently converting said obtained hydrogen sulfide into biomass by means of sulfide oxidizing bacteria (SOB). In particular, said conversions are mediated by electron transfer of one or more energy rich gases.

As indicated before, energy rich gases are rich in chemical energy. Non-limiting examples are hydrogen, CO, H₂S, PH₃, CH₄, volatile organics, mixes of such gases, gases produced by fermentation such as H₂ and biogas, or by physical/chemical gasification processes such as syngas made from fossil or from renewable resources or wastes. Such energy rich gases can serve as electron donors, and can give rise to the generation of added value microbial products by means of biochemical conversions in which these electrons are transferred to electron acceptor compounds.

In an embodiment, said energy rich gases are selected from hydrogen, CO, H₂S, PH₃, CH₄, volatile organics, gases produced by fermentation, and mixes of such gases.

In a preferred embodiment, said energy rich gas is hydrogen.

Said sulfate reducing microorganisms can be any microorganisms which are able to reduce sulfur compounds to hydrogen sulfide. In an embodiment, said sulfate reducing microorganisms which convert a sulfur compound to hydrogen sulfide are selected from sulfate reducing bacteria (SRB) and/or sulfate reducing archaea (SRA).

Examples of such sulfate reducing bacteria (SRB) include: *Desulfovibrio sp.* such as, but not limited to, *Desulfovibrio vulgaris* (Marburg, Madison or Hildenborough), *Desulfovibrio* G11, *Desulfovibrio desulfuricans* (Essex 6); *Desulfotomaculum orientis; Desulfobacterium autotrophicum; Desulfobulbus sp.; Desulfotomaculum sp.; Desulfosporomusa sp.; Desulfosporosinus sp.; Thermodesulfovibrio sp. and Geobacter sp..*

Examples of such sulfate reducing archaea (SRA) include: *Archaeoglobus sp.; Thermocladium sp. and Thermodesulfobium sp..*

It will be understood that also combinations of different microorganisms can be applied, and/or that a consortium or a mixture of microorganisms comprising at least one SRB and/or at least one SRA can be applied.

Said microorganisms can be naturally occurring or may be genetically modified, e.g. by mutagenesis or by the introduction of foreign plasmids, genes or gene fragments.

In an embodiment of the invention, the conversion of said sulfur compound to hydrogen sulfide occurs under anaerobic conditions.

In another or further embodiment, the reduction of said sulfur compound occurs in the presence a carbon dioxide source.

Said sulfur compound to be converted to hydrogen sulfide may be any oxidized sulfur compound, such as, but not limited to, sulfate (SO₄²⁻), sulfite (SO₃²⁻), dithionite (S₂O₄²⁻), thiosulfate (S₂O₃²⁻), trithionate (S₃O₆²⁻), tetrathionate (S₄O₆²⁻), elemental sulfur (S₈), and polysulfides (Sₙ²⁻). In a preferred embodiment, said sulfur compound is sulfate or thiosulfate.

Sulfate reducing microorganisms, such as SRB and SRA are very special in the sense that about any type of bioavailable and biodegradable electron donor compound can be metabolized by SRA or SRB (Qian et al. 2019). They can be divided into heterotrophic and autotrophic sulfate reducing microorganisms. Heterotrophic ones obtain energy for growth from organic substrates. Autotrophic ones use CO₂ as carbon source and obtain electrons from oxidation of H₂ (Lens & Kuenen, 2001). Many incompletely (such as *Desulfovibrio sp*.) and completely (such as *Sulfosarcina variabilis, Desulfonema limicola, Desulfococcus niacini and Desulfobacterium autotrophicum*) hydrogen oxidizing sulfate reducers can grow on H₂ as sole energy source (Widdel 1988; Colleran et al., 1995). Hydrogenotrophic sulfate reduction, or sulfidogenesis, involves:

4H₂ + SO₄²⁻ + H⁺ → HS⁻ + 4H₂O, ΔG = -38kJ/mol [1]

This reaction is feasible only in the absence of oxygen and nitrate. SRB prefer an environment about pH=7 and are usually inhibited at pH values below 5.5 or above 9. The optimum temperature for most pure cultures of SRB ranges between 28-32 °C. (Hao, 2003). When H₂ is used as an electron donor, CO₂ or bicarbonate must also be present to supply carbon for the SRB. Addition of CO₂ can lead to a decrease in the pH of the system because of the formation of carbonic acid. Consumption of H₂ from SRB leads to hydroxide formation [2], resulting in pH increase (Liamleam & Annachhatre, 2007).

8H₂ + 2SO₄²⁻ → H₂S + HS⁻ + 5H₂O + 3OH⁻ [2]

Under anaerobic conditions, hydrogen can be used as substrate by both sulfate reducing microorganisms and methanogenic bacteria (MB). When there is an excess of sulfate in the reactor, H₂ is mainly used by the sulfate reducing microorganisms (Visser et al., 1993). According to [1] and [2], the free energy ΔG of sulfate reduction using hydrogen as electron donor is slightly more favorable compared with that for methanogens (MB):

4H₂ + CO₂ → CH₄ + 2H₂O, ΔG = - 32kJ/mol [3]

In addition, Monod (growing) and Michaelis-Menten (resting) kinetic parameters have been calculated for different bacteria strains from both types, indicating that sulfate reducing microorganisms are better equipped to take up H₂ than MB (Robinson & Tiedje, 1984). Sulfate reducing microorganisms can effectively utilize hydrogen at lower partial pressure than methanogens (Lovley etal., 1982). Thus, when sulfate is in excess and hydrogen is the limiting factor, SRB will consume H₂ to a concentration that is too low to allow methanogens to be active (Lens & Kuenen, 2001).

SRB are strictly anaerobes but they can survive under oxygen exposure. The critical dissolved oxygen concentration (DO) in the water below which sulfate reduction can occur is 0.1-1.0 mg/L. DO above 1.0 mg/L is inhibitive for sulfate reducers (EPA,1985).

Biological sulfate reduction using synthesis gas (H₂, CO and CO₂) as an electron donor and carbon source allows a sulfate conversion rates to 10 g COD-H₂ equivalent/L.day having a biomass concentration of 2.6 g/L (Van Houten et al., 1996). The biomass yield (in g cell dry weight (CDW)/mol H₂) for SRB is of the order of 1.9 g CDW/mol H₂ which corresponds with 0.12g CDW/g COD-H₂ converted (Liamleam & Annachhatre, 2007;Speece, 1996). Kinetic characteristics were measured for many SRB hydrogenotrophic strains (Table 1) using sulfate as electron acceptor.

**Table 1: Kinetic characteristics of hydrogenotrophic SRB strains growing on H₂ and sulfate as sole energy source**

| **Organism** | **µₘₐₓ (h⁻¹)** | **Y (CDW/g COD-H₂)** | **Reference** |
|---|---|---|---|
| *Desulfovibrio vulgaris* (Marburg) | 0.15 | 0.13 | Badziong & Thauer, 1978 |
| *Desulfovibrio* G11 | 0.05-0.07 | 0.09-0.13 | Robinson &Tiedje, 1984 |
| *Desulfovibrio vulgaris* (Madison) | 0.035 | 0.05-0.08 | Lupton & Zeikus, 1984 and Badziong, Thauer, & Zeikus, 1978 |
| *Desulfovibrio desulfuricans* (Essex 6) | 0.15 | 0.12 | Brandis & Thauer, 1981 |
| *Desulfovibrio vulgaris* (Hildenborough) | 0.2 | 0.15 | Brandis & Thauer, 1981 |
| *Desulfotomaculum orientis* | 0.09 | 0.19 | Cypionka & Pfennig, 1986 |
| *Desulfobacterium autotrophicum* | 0.04-0.07 | - | Brysch et al., 1987 |

Said sulfur compounds, which are converted to hydrogen sulfide by sulfate reducing microorganisms, can be found in the environment in all forms, but mostly as solids. In seawater sulfate ion is the most abundant ion after chloride. In freshwater, sulfate ion is the most abundant ion after carbonate (Bharathi, 2008). Under aerobic conditions, sulfate is the thermodynamically stable form of sulfur, whereas hydrogen sulfide is the stable form under anaerobic conditions. When sulfides are dissolved in water in absence of oxygen they are present as bisulfide (HS⁻), sulfide ions (S²⁻), and hydrogen sulfide (H₂S) which is volatile. These forms coexist in equilibrium and their distribution depends on the pH. Above pH 8-9, virtually all dissolved sulfide is present in the ionized form (HS⁻). At neutral pH values, approximately 20-50% of the dissolved sulfide is present in H₂S form (Colleran et al., 1995) (Figure 1). The escape of H₂S gas depends on the degree of mixing and surrounding ambient H₂S gas concentration. Its presence in gaseous form can easily be recognized by a distinctive rotten-egg smell.

Many studies have shown that hydrogen sulfide (H₂S) produced from sulfate reduction can be toxic for a variety of bacteria at low concentrations and also for the SRB at high concentrations. Sulfide inhibits SRB probably when the sulfide species (H₂S, HS⁻ or S²⁻) react with the iron of ferredoxin, cytochromes or other essential metal compounds for bacterial metabolism (Hao, 2003; Okabe, et al., 1992). It was found that at about 500 mg/L of total sulfide, 50 % inhibition of lactate utilization by *D. desulfuricans* occurs (Okabe et I., 1992). A hydrogen sulfide concentration of 547 mg/L (16.1 mM) completely inhibited a SRB culture growth (Reis et al., 1992). In a recent study, cells of *Desulfovibrio vulgaris* were grown under high sulfide accumulation (10 mM) and under low sulfide concentration (1 mM). It was determined that high sulfide decreased the growth rate constant by 52 % and the final cell density of the culture 33 %. It was indicated that high sulfide represents a stress condition for SRB, in which the bioavailability of metals may be lowered. Overall levels above 5 mM of H₂S can give rise to a decreased growth rate and less efficient biomass production (Caffrey & Voordouw, 2010).

The sulfate reducing microorganisms of present invention are preferably those organisms capable of reducing sulfur compounds such as, but not limited to, sulfate and thiosulfate, and which can use one or more energy rich gases as electron donor for the conversion of said sulfur compound in (hydrogen) sulfide or other energy rich metabolites/products or energy dense metabolites/products such as, but not limited to, phosphates liberated by sulfides, phosphines, ammonium, amines, amino acids, proteins, chain elongations products, long chain fatty acids, sulfur granules and/or waxes. All are examples of products derived from the initial gases, representing a densification of the original chemical.

As indicated before, energy rich gases are rich in chemical energy. Non-limiting examples are hydrogen, CO, H₂S, PH₃, CH₄, volatile organics, mixes of such gases, gases produced by fermentation such as H₂ and biogas, or by physical/chemical gasification processes such as syngas made from fossil or from renewable resources or wastes. Such energy rich gases can serve as electron donors, and can give rise to the generation of added value microbial products by means of biochemical conversions in which these electrons are transferred to electron acceptor compounds.

"Densification" in the context of the present invention relates to an increase in amount of Chemical Oxygen Demand (COD) per unit volume (mL, L) of the compound.

"Chemical oxygen demand" is an indicative measure of the amount of oxygen that can be consumed by reactions in a measured solution. It is commonly expressed in mass of oxygen consumed over volume of solution which in SI units is milligrams per liter (mg/L). A COD test can be used to easily quantify the amount of organics in water.

The energy rich gases can be at the onset of all previously described electron dense (depository) compounds or energy rich metabolites/products (hydrogen sulfide or other energy rich metabolites/products or energy dense metabolites/products such as, but not limited to, phosphates liberated by sulfides, phosphines, ammonium, amines, amino acids, proteins, chain elongations products, long chain fatty acids, sulfur granules and/or waxes; as indicated before), this by clever selection of the proper microbial cell factory mechanisms, be it in a single strain of in combinations thereof. Conversion to energy rich metabolites/products can be performed by the sulfate reducing microorganisms alone or in association with partner-microorganisms. By using organisms specially equipped to metabolize energy rich gases, the rates of formation and yields of the overall process to generate microbial products become of increased industrial interest.

The present method is advantageous in that the functional group of microorganisms capable to reduce sulfur compounds as sulfate and thiosulfate is specially equipped to deal with the energy rich electrons donor gases such as those mentioned above and with hydrogen in particular.

The conversion of the gaseous electron donors to the subsequent liquid or soluble energy rich products allows to provide a subsequent fermentation-conversion step in which the metabolites of the sulfate reducing microorganisms are used by other bacteria thriving on these energy dense products.

As indicated before, said hydrogen sulfide and/or other energy rich/dense products obtained from the conversion of sulfur compounds by sulfate reducing microorganisms, is/are subsequently converted into biomass or biomass derivatives by means of sulfide oxidizing bacteria (SOB).

In an embodiment, said biomass derivatives are chosen from but not limited to proteins, single-cell protein, polyhydroxy butyrate and analogues, polyphosphates, carbohydrates, cell wall components such as but not limited to exopolysaccharides in general and sulfated polysaccharides in particular, lipids, extracellular dissolved organic carbon compounds, and metabolites.

Examples of such sulfide oxidizing bacteria (SOB) include: *Thiobacillus sp.; Thiothrix sp.; Halothiobacillus sp.; Acidithiobacillus sp.; Thioalkalivibrio sp.; Thiomicrospira sp.; Thermothrix sp.; Chlorobaculum sp.* and *Beggiatoa sp..*

It will be understood that also combinations of different microorganisms can be applied, and/or that a consortium or a mixture of microorganisms comprising at least one SOB can be applied.

Said microorganisms can be naturally occurring or may be genetically modified, e.g. by mutagenesis or by the introduction of foreign plasmids, genes or gene fragments.

In an embodiment, said converting of hydrogen sulfide into biomass and derivatives occurs under aerobic conditions and thus in the presence of oxygen; or using nitrate. In this reaction, hydrogen sulfide is oxidized to sulfate or thiosulfate, and the electron donor, H₂, is oxidized to H₂O.

Various types of species growing or the densified products of the original energy rich gases can be utilized but the typical ones are the sulfide oxidizing bacteria (SOB). They exist in two classes: the ones which grow with light and without oxygen, and the ones which grow with oxygen. The first group grows at neutral pH and can reach cell yields of the order of 1,0 g biomass formed per g COD- electron equivalent used (in the form of H2S).The latter can grow in the pH range of 1-8 and in the temperature range of 5-60 °C. (Shivvers and Brock 1973 ;Sublette 1988). Yields of the order of 0,1 g cell dry weight per g COD-H₂S have been reported by Sublette (1988) for autotrophic growth.

Thermodynamically, growth on hydrogen and oxygen in one step follows the equations [4],[5]. These reactions give a total ΔG=-118 kJ/mol electron equivalent:

½ H₂ → H⁺ + e⁻, ΔG = -40 kJ/mol [4]

¼ O₂ + H⁺ + e⁻ → ½ H₂O, ΔG = -78 kJ/mol [5]

In a preferred embodiment, the oxidation of the energy rich gas, as an example hydrogen, occurs in two steps. First, anaerobically sulfate reducing microorganisms use hydrogen to reduce sulfate to sulfide. This gives a total ΔG=-21 kJ/per e⁻ equivalent for sulfate reducing microorganisms.

1/8 SO₄²⁻+ 19/16 H⁺ + e⁻ → 1/16 H₂S + 1/16 HS⁻ + ½ H₂O, ΔG = - 21 kJ/mol [6]

Combined with [4], this results in -40 kJ/mol - 21 kJ/mol = -61 kJ/mol

At the second step the electron dense sulfide is oxidized back with oxygen (or nitrate respectively nitrite) to sulfate (or thiosulfate) by sulfide oxidizing bacteria (SOB). This cycle of the S-species corresponds with the oxidation of the electron donor (H₂) via H₂S by oxygen to water (H₂O).

This second step consists of [4], [5] and [6] equations in reverse, having a total ΔG= -118 - (-61) = -57 kJ/mol electron equivalent. The total potential energy of the equations remains ΔG = -118.48 kJ/mol. Hence the sulfate reducers get only about 50 % (57/118) of the potential energy for their biomass production, while sulfide oxidizers get the remaining 50 %. SOB are known to use mineral nitrogen (ammonium, nitrate) to grow, but some species can also fix dinitrogen and convert it to ammonia respectively protein nitrogen. The fact that one can use the powerful catalytic capability of the SRB, and this at the cost of only a limited amount of the potential overall energy gain, is a novel finding and at the basis of this development.

The difficulty of fermenting gases is that they are generally poorly soluble in water. Yet, the microbial processes should proceed in aqueous environmental conditions. A limiting factor is that the gases have to be taken into the microbial cells in order to become part of the metabolic pathways. A key feature of this invention is that it particularly focuses on enzymes which have been selected for their natural processes and to be able to take up gaseous energy compounds with high rate and high affinity. For instance, various types of hydrogenases are described for a great variety of organisms. According to Cord-Ruwisch et al. 1988 (Table 2), a high affinity for hydrogen with inorganic electron acceptors is demonstrated by Desulfovibrio and Wollinella. The paper of Cord-Ruwish et al. 1988 demonstrates that for H₂ as an electron donor, when sulfate is present, the sulfate reducing microorganisms reach by far the lowest residual hydrogen level, about some 5 ppm, while for methanogens this is about factor 3 higher and homoacetongens even about factor 50 higher.

**Table 2: Effect on electron acceptor on the threshold of H₂ in different hydrogenophilic bacteria growing on organic substrates of H₂. Values given are means of at least duplicate experiments. Reproducibility was about 50 %. ^{a} D. fructosovorans disproportionates fumarate to acetate and succinate, so that fumarate as electron acceptor may become limiting at the end of growth rather than H₂.**

| Electron acceptor oxidized/reduced | Microorganism | Substrate | H₂ [ppm] |
|---|---|---|---|
| CO₂/acetate | *Sporomusa acidovorans* | methanol | 430 |
| | *Sporomusa termitida* | H₂ | 830 |
| | *Acetobacterium woodii* | H₂ | 520 |
| | *Acetobacterium carbinolicum* | H₂ | 950 |
| Sulfur/sulfide | *Desulfovibrio fructosovorans* JJ | lactate | 24 |
| | *Desulfovibrio desulfuricans* Essex | lactate | 10 |
| | *Wolinella succinogenes* | H₂ | 5 |
| CO₂/CH₄ | *Methanospirillum hungatei* | formate | 25 |
| | *Methanospirillum hungatei* | H₂ | 30 |
| | *Methanobrevibacter smithii* | H₂ | 100 |
| | *Methanobrevibacter arboriphilus* | H₂ | 90 |
| | *Methanobacterium formicum* | H₂ | 28 |
| | *Methanococcus vannielii* | H₂ | 75 |
| Sulfate/sulfide | *Desulfovibrio fructosovorans* JJ | lactate | 12 |
| | *Desulfovibrio vulgaris* Hildenborough | lactate | 19 |
| | *Desulfovibrio vulgaris* G6 | lactate | 16 |
| | *Desulfovibrio desulfuricans* Essex | lactate | 8 |
| | *Desulfovibrio desulfuricans* Essex | H₂ | 9 |
| | *Desulfobulbus elongatus* | lactate | 13 |
| Sulfite/sulfide | *Desulfovibrio desulfuricans* Essex | lactate | 6 |
| Thiosulfate/sulfide | *Desulfovibrio desulfuricans* Essex | lactate | 7 |
| Caffeate/hydrocaffeate | *Acetobacterium woodii* | H₂ | 3 |
| Fumarate/succinate | *Wolinella succinogenes* | H₂ | 0.02 |
| | *Desulfovibrio fructosovorans* JJ | fumarate | 0.9^{a} |
| Nitrate/ammonia | *Desulfovibrio desulfuricans* | lactate | 0.03 |
| | *Desulfovibrio desulfuricans* | H₂ | 0.03 |
| | *Wolinella succinogenes* | H₂ | 0.02 |

It must be noted that in overall energy flow between microorganisms, it is conceivable that conditions created by the SRMs can still be of such nature that they allow the co-occurrence of other species and thus permit end products other than only sulfide. Thus it can be possible that the SRMs are provoking species such as homoacetogenic bacteria and chain-elongating bacteria to produce metabolites such as short and long chain fatty acids and even waxes.

Overall, the free energy change of the reaction of hydrogen with oxygen to give water is subdivided by two sets of organisms. The first set are the sulfate reducers, having as anaerobes a low cell yield of the order of 0,1 g cell dry weight (CDW) per g of COD-H₂ formed and thus fixing a relatively limited amount of CO₂ to produce microbial products. The second set are the sulfide oxidizers. The latter when they grow autotrophically have a growth yield typical for all chemo- autotrophs i.e. in the order of 0,1-0,2 g CDW per g COD- H₂S converted. To grow and thus form biomass, they can fix CO₂ or use organic carbon molecules. To get a detailed view of the energy efficiency of the S oxidizers, see Klatt and Polerecky 2015.

H₂S + 1,65 O₂ + 0,65 CO₂ + 0,35 H₂O --> SO₄²⁻ + 0,35 CH₂O + 2H⁺

(wherein CH₂O represents the biomass formed)

It is well known that sulfide can also be taken up by phototrophic bacteria achieving high cell yields of about 1,0 g cell dry matter formed per g of COD subjected to their phototrophic metabolism (Saer & Blankenship 2017; Garcia et al.2017). However, the need to supply light is a factor of concern in the overall application of this process.

Hence for the chemo-autotroph: 4 moles H₂ correspond with the formation of 1 mol H₂S and the latter generates:

0,35x 30 g =10,5 g biomass CH₂O

4x 16 = 64 g COD-H₂ generate 10,5 g biomass.

The theoretical maximum yield of the S-oxidizer is: 10,5 g Cell dry weight/64 g COD-H₂ = 0,16 g CDW/g COD-H₂ input when using H₂S.
Effectively, under conditions of low dissolved oxygen and low sulfide, a yield of 0,13 g CDW per g COD-H₂S was reported by Nelson et al. 1986.

Of course, synergistic effects such as the stimulation of aerobic hydrogenotrophs by sulfide has been described by Suylen et al. 1986 (Table 3). Thus, it is conceivable that co-culturing of sulfate reducers and hydrogenotrophs can be engineered and yield technical industrial advantages.

**Table 3: yield data on Hyphomicrobium EG. Dry weight, protein and total organic carbon of Hyphomicrobium EG grown mixotrophically on methylamine (MA) and increasing concentrations of thio(sulfate) and sulfide (S2-) at D = 0.035 h-1. All cultures were MA limited. In the MA plus sulfide cultures sulfide was not detectable.**

| Substrate | Dry weight (mg l⁻¹) | Protein (mg l⁻¹) | Total organic carbon (mg l⁻¹) | Residual thio in culture (mM) |
|---|---|---|---|---|
| 10 mM MA | 108 ± 3 | 47 ± 4 | 51 ± 2 | 0 |
| 10 mM MA + 1.1 mM thio | 116 ± 6 | 42 ± 4 | 69 ± 1 | 0.26 ± 0.04 |
| 10 mM MA + 5.5 mM thio | 144 ± 1 | 56 ± 3 | 72 ± 1 | 1.10 ± 0.04 |
| 10 mM MA + 8.55 mM thio | 165 ± 3 | 68 ± 2 | 85 ± 1 | 1.65 ± 0.05 |
| 9.8 mM MA + 0.93 mM S²⁻ | 116 ± 7 | 46 ± 6 | 56 ± 1 | 0 |
| 9.8 mM MA + 5.4 mM S²⁻ | 152 ± 3 | 55 ± 1 | 73 ± 1 | 0.08 ± 0.04 |
| 9.5 mM MA + 10.4 mM S²⁻ | 212 ± 3 | 77 ± 2 | 96 ± 1 | 0.11 ± 0.05 |

In an embodiment of the invention, the conversion reaction wherein the sulfate reducing microorganisms convert sulfur compounds to hydrogen sulfide occurs at a pH of between 4 and 10, preferably between 4 and 9.

Preferably, at the end of the reaction, the pH is in general about 7 to 8.

In an embodiment, the conversion reaction wherein said hydrogen sulfide is subsequently converted into biomass and derivatives by means of sulfide oxidizing bacteria occurs at a pH of between 3 and 9, preferably between 3.5 and 8.5.

Preferably, at the end of the reaction converting hydrogen sulfide into biomass and derivatives, the pH is in general about 6 to 7.5, or in general 0.5-1 lower than at the end of the reaction converting of sulfur compounds to hydrogen sulfide, and could even be lower as the acid H₂SO₄ may be produced.

In an embodiment of the present invention, said reactions of converting a sulfur compound to hydrogen sulfide and subsequently converting said hydrogen sulfide into biomass and derivatives occur in a bioreactor.

Different types of bioreactors are known in the art, and a person skilled in the art will understand which types can be used for said conversion reactions. As non-limiting examples, the following bioreactors may be used: continuous stirred tank reactor (CSTR), trickle-bed reactor (TBR), a moving bed bioreactor and/or membrane reactor(s). Also, the method can be applied in a batch reactor or continuous reactor or a combination of a batch reactor and continuous reactor. In case the reactor involves a continuous culture, biomasses is preferably retained by membrane or in granules, floes or biofilms, optionally involving additional materials for immobilization of the biomass. The different processes that are illustrated can be performed in different compartments or in different reactors.

(Energy dense) gas mixing in the bioreactor will also depend on the type of bioreactor, and can for example be fine gas bubble mixing under low to high pressure or jet mixing. More information about gas-liquid transfer can be obtained from Yasin et al. 2015.

In an embodiment, said conversion reactions occur in separate compartments of a bioreactor, or separate bioreactors which are in (fluid) connection with each other. As a non-limiting example, the conversion of sulfur compounds to hydrogen sulfide may be performed in a trickle-bed reactor, while the conversion of hydrogen sulfide to biomass may be performed in a continuous stirred tank reactor (CSTR).

In another embodiment, said conversion reactions occur in the same compartment of a bioreactor. Preferably, both reaction are still separated from each other, such as separated by reactor conditions or by diffusion gradients. As a non-limiting example for the latter, both conversion reactions can occur in microbial granular systems or in reactors equipped with membrane systems to keep them separated.

In an embodiment, the conversion reactions occur in aqueous environmental conditions.

"Aqueous environmental conditions" refers to a condition or state of an environment, in which the environment comprises water, and/or wherein the environment is thus humid. Examples of such environments include process water, manure, composts and sludges.

In an embodiment, waste material is used as starting material for the conversion reactions of the current method. This waste material can be any type of waste, preferably biowaste, preferably waste material comprising a sulfur compound.

"Biowaste" or "biodegradable waste" includes any organic matter in waste which can be broken down into carbon dioxide, water, methane or simple organic molecules by microorganisms and other living things by composting, aerobic digestion, anaerobic digestion or similar processes. It further includes some inorganic materials which can be decomposed by bacteria. Such materials include gypsum and its products such as plasterboard and other simple organic sulfates which can decompose to yield hydrogen sulfide in anaerobic conditions. Other non-limiting examples of (bio)waste include soils, composts, waste water, industrial process water, (sewage) sludge, manure, ...

In an embodiment, this waste material is an industrial side stream or industrial coproduct. Industrial side streams or coproducts may be obtained by the industrial processing of biological materials, which generates process water that is rich in organic compounds, such as rich in protein, carbohydrate and/or fat. They may also include various minerals. Non-limiting examples of industrial side streams are wastewater, process water, sewage sludge from the agroindustry or urban wastewater.

In an embodiment, an enrichment culture of waste material, as described above or below, is used as starting material for the conversion reactions of the current method. Preferably, these are enrichment cultures of biowaste material, preferably of waste material comprising a sulfur compound.

Preferably, the waste, biowaste or waste comprising a sulfur compound is waste derived from the agroindustry, such as from processing of organic material, biological and preferably vegetable material.

This may also be from another industrial process, preferably there is no material in the waste that is inadmissible for the production of feed or food from the biomass; or inadmissible for the production of feed and/or food from biomass derivatives, or inadmissible for biomass derivatives to be used in agricultural, nutritional, cosmetic and/or pharmaceutical applications or as biopolymer.

Preference is given to ensuring that the supplied waste does not contain any harmful components that could inhibit or stop the growth of the microorganisms.

Additives and/or additives, such as nitrogen (N), phosphorus (P), trace elements and vitamins, can preferably be added to the supply of waste, resulting in an optimal growth and multiplication of the microorganisms and a maximum production of the desired biomass derivatives. The additives can also apply special growth conditions for desired micro-organisms in terms of surface tension, viscosity and additional metabolic energy supply.

The temperature of the processed waste in the reactor is preferably brought to a value between 10 and 70 °C, preferably between 20 and 50 °C, preferably between 25 and 50 °C, more preferably between 27 and 40 °C, more preferably between 27 and 35 °C, more preferably between 28 and 32 °C and preferably about 30 °C.

The pH value in the SRM reactor (compartment) wherein the sulfate reducing microorganisms convert sulfur compounds to hydrogen sulfide is preferably brought to a value between 4 and 10 and more preferably between 4 and 9, and preferably by adding a buffer solution, an acid or a base.

The pH of the effluent of said SRM reactor (component) is in general about 7 to 8.

The pH value in the SOB reactor (component) wherein said hydrogen sulfide is subsequently converted into biomass and derivatives by means of sulfide oxidizing bacteria is preferably brought to a value between 3 and 9, preferably between 3.5 and 8.5.

The pH of the effluent of said SOB reactor (component) is in general about 6 to 7.5, or in general 0.5-1 lower than that of the SRM bioreactor (component) and could be even lower as the acid H₂SO₄ can be produced.

In this document, the term "buffer" refers to an aqueous solution of two substances, i.e. buffer substances, which are in a certain equilibrium and take on a certain pH. When diluting, adding an acid or a base, this pH will remain almost constant. The disturbance of the equilibrium and the acidity is thus 'buffered'. Buffer solutions preferably consist of an acid/base couple; either an acid and the salt of its conjugated base, or a base and the salt of its conjugated acid. Both are preferably weak acids or bases.

In a preferred embodiment, the method relates to a method for producing biomass or derivatives thereof, from material comprising one or more sulfur compounds, comprising the following steps:
- converting said sulfur compound(s) to hydrogen sulfide by sulfate reducing microorganisms under anaerobic conditions; and subsequently
- converting obtained hydrogen sulfide by into biomass by means of sulfide oxidizing bacteria under aerobic conditions;
wherein said conversions are mediated by energy rich gases, and said conversions take place in one or more bioreactors.

In another of further embodiment, the hydrogen sulfide conversion reaction occurs in open systems such as soils, composts, activated sludges, and other biotic or abiotic fermentation systems; and thus not in a previously described (bio)reactor.

"Fermentation" is the chemical breakdown of a substance by, in general, microorganisms, and whereby energy is released. As already indicated before, it is known that energy rich gases can serve as electron donors and, by means of biochemical conversions in which these electrons are transferred to proper electron acceptor compounds, can give rise to the generation of added value microbial biomass and/or microbial products, using fermentation reactions.

From the reaction of converting sulfur compounds to hydrogen sulfide, microbial biomass can be collected which is rich in enzymes capable to metabolize gaseous energy rich compounds, microbial biomass capable to abate iron corrosion by bringing forward ferric reduction and densification (Koolie et al. 2019), products such as reduced iron, sulfur, sulfides and polysulfides/short chain and long chain fatty acids and waxes (Wahlen et al.2009), phosphates liberated by sulfides, phosphines, ammonium, amines, amino acids, proteins, chain elongations products and/or sulfur granules.

The fermentation liquor of the bioreactor (compartment) of the conversion of sulfur compounds to hydrogen sulfide contains reduced compounds of which some have the capacity to influence various microbial processes (aerobic and anaerobic). The result of the densification of the energy rich gases by means of the sulfate reducing microorganisms can be used to modify microbial conversions as such in complex matrices as for instance soils, activated sludge, digesters, composts and other biotic of abiotic fermentation systems, preferably using sulfate oxidizing bacteria.

From the conversion of hydrogen sulfide to biomass, microbial biomass can be collected rich in proteins such as single-cell protein, polyhydroxyalkanoate (PHA), various forms of extracellular polymeric substances such as sulfated polysaccharides, poly phosphates, compounds specific for thiobacilli such as sulfur (Janssen et al.2001) or amphipathic membrane vesicles (Knickerbocker et al. 2000; Zhang et al. 2009), extracellular dissolved organic carbon which might inhibit certain classes of bacteria (Wang et al.2018), waxes and more.

When biomass is produced in one or more bioreactors, said biomass preferably is harvested from the reactor in a way that guarantees the quality of the biomass and derivatives formed.

The harvesting of the produced biomass is preferably done by sedimentation, flotation, filtration and/or centrifugation to obtain a paste with preferably a density between 1 and 50 %, or between 5 and 25 % by weight of dry biomass. It is essential that the paste is protected from contamination by other undesired micro-organisms and from spoilage of the proteins present. If necessary, a pasteurization or cooling step is provided.

In a preferred form, the harvested biomass is post-treated, whereby one step of this post-treatment is the killing of the microorganisms without reducing the quality of their cell proteins. This can be done in one of the ways mentioned in the non-exclusive list of pasteurization, steam treatment, irradiation, chemical methods or mechanical stress. During this step or afterwards, the paste can be subjected to drying or extrusion with preservation of the qualitative properties of the derivatives in or on the biomass, after which the derivatives can possibly be processed.

In a particularly preferred embodiment, said method of the current invention allows the production of sulfated polysaccharides, said method comprises the following steps:
- converting sulfur compound(s) to hydrogen sulfide by sulfate reducing microorganisms under anaerobic conditions; and subsequently
- converting obtained hydrogen sulfide by into biomass by means of sulfide oxidizing bacteria under aerobic conditions;
wherein said conversions are mediated by energy rich gases, and said conversions take place in one or more bioreactors.

Said sulfated polysaccharides may be chosen from the group of heparin, heparan sulfates, fucoidan, glycosaminoglycans, carrageenans, agar, ulvans and mixtures thereof.

In an embodiment, the biomass or derivatives, may subsequently be used in feed (fish feed, animal feed, pet food) and/or food for human consumption and/or as slow release organic fertilizer. Biomass derivatives may have applications in feed and/or food, in agricultural, nutritional, cosmetic and/or pharmaceutical applications or as biopolymer.

The method of producing biomass or derivatives thereof of the present invention thereby contributes to the economical optimal use of nutrient raw materials.

In another aspect, the present invention concerns a bioreactor system for producing biomass or derivatives thereof, using sulfate reducing microorganisms and sulfide oxidizing bacteria, the system comprising:
- a first compartment comprising at least one sulfate reducing microorganism culture, one gas inlet configured to supply an amount of energy rich gas, preferably at least one gas outlet, at least one fluid inlet, and at least one fluid outlet;
- a second compartment comprising at least one sulfide oxidizing bacteria culture.

In an embodiment, the first compartment and the second compartment of the system are both separate bioreactors, preferably in fluid connection with each other.
In another embodiment, the first compartment and the second compartment of the system are separate compartments in the same bioreactor, preferably in fluid connection with each other.

The fluid inlet of the first compartment can provide a fluid, preferably a fluid with a sulfur compound, preferably a waste such as biowaste, most preferably a biowaste comprising a sulfur compound, into the first compartment. This fluid is then is allowed to contact the sulfate reducing microorganisms.

Preferably, the first compartment comprising the sulfate reducing microorganisms is configured to operate under anaerobic conditions. Preferably, the second compartment comprising the sulfide oxidizing microorganisms is configured to operate under aerobic conditions.

The sulfate reducing microorganisms are able to use the energy rich gas to convert a sulfur compound to hydrogen sulfide.

In the second compartment, such hydrogen sulfide can be converted into biomass and derivatives by means of sulfide oxidizing bacteria.

In an embodiment, the system comprises one or more harvesting elements, which are capable of harvesting the produced biomass and derivatives. Such harvesting elements may be suitable for harvesting biomass by any method known in the art. Preferably, said elements are suitable for harvesting via sedimentation, flotation, filtration and/or centrifugation.

Preferably, said harvesting element(s) is/are connected to the second compartment of the bioreactor system. More preferably, between the second compartment and the harvesting element(s), is a harvesting port, which can be closed during biomass production and opened to harvest biomass.

Preferably, the harvested biomass can be collected in a collection tank, which is in connection with the second compartment of the system, preferably connected via said harvesting element(s).

A person of ordinary skill in the art will appreciate that elements of the aspect of the method as described above return in the aspect of the system of the invention. Consequently, all aspects of the present invention are related. All features and advantages as described in one of the aspects, as described above as well as below, can relate to any of these aspects, even if they are described in conjunction with a specific aspect.

In an embodiment, the system of the present invention is capable of performing the method of one of the embodiments of the invention.

In an embodiment, sulfur reducing microorganisms, archaea or bacteria (SRB: Desulfo, Desulfuro and similar species but also Geobacter species and other sulfate reducers) are used as industrial organisms for densification of energy rich gases, by preference under low or atmospheric reactor conditions, producing energy rich products. These products are used as such, or they can be in the same or in a second reactor phase further converted by microorganism operating the aerobic loop of the sulfur cycle (SOB). These sulfur oxidizing bacteria (SOB) work alone or in combination with other organisms and bring forward metabolism yielding valuable products ranging from biomass to biomass derivatives to metabolites such as mineral and organic nitrogen, organic polymers, sulfur and phosphate.

Possible embodiments of the invention may be:
1. Valuable products generated by means of the implementation of the combination of the reductive and the oxidative part of the sulfur cycle such as various types of biomasses of sulfur reducing bacteria, sulfur oxidizing bacteria, combinations of the latter biomasses and metabolic products of these and the latter combinations.
2. Microbial based systems using enzymes with very high affinity for CO, H₂ and CH₄ as naturally available in the groups of bacteria which can reduce thiosulfate respectively sulfate either by direct or by mediated electron transfer and which typically are capable to convert the latter electron donors to valuable electron dense products such as sulfide, sulfur and organic compounds such as acetate or higher reduced organic compounds such as long chain fatty acids and waxes. The latter compounds can subsequently under conditions of higher redox level be upgraded generally in combination with CO₂ fixing by chemolithotrophic or phototrophic microorganisms, but also by mixotrophic organisms, to products such as microbial biomass and various microbial metabolites such as sulfur, sulfate, protein, exocellular organics, phosphate...
   The key feature: Use of SRB (Desulfo, Desulfuro and similar species but also Geobacter species and other sulfate reducers) as industrial organisms for densification of energy rich gases which then in a second phase can advantageously be further converted by microorganism operating the second loop of the sulfur cycle or any advantageous metabolism.
3. Microbial based systems as described above, present in pure or mixed culture, which can serve as pacemaker for other groups of fermentative enzymes and bacteria which upgrade the electron dense products of the first group to other products even at low redox conditions. Examples are homoacetogens growing next to the group of sulfate reducing bacteria and associated species capable in the presence of sufficient energy rich gas as electron donor to form waxes. Another example is aerobic mixotrophs capable to operate in high sulfide/sulfate environments and produce from soluble COD valuable composites and EPS.
4. The systems as described above in which the actuators being the microorganisms are separated in time or space in order to optimize the quality and/or rate of production of the end products.
5. The systems as described above in which the actuators being the microorganisms are all growing together in one reactor configuration.
6. The systems as described above operating but with use of oxidized nitrogen species (nitrite/nitrate) or light or electrogenesis as a driver of the upgrading of the intermediates formed by the SRB.
7. The systems as described above but in which the second step is implemented not in reactor but in open systems such as soils, composts, activated sludges, and other biotic or abiotic fermentation systems.
8. The combination of SRB as all-round transformers of various types (solid, dissolved, dispersed, gaseous) metabolizable compounds to products which subsequently can be upgraded by SOB and associated bacteria to valuable compounds.
9. A novel method based on combining the reductive and the oxidative loop of the sulfur cycle by making use of use of low value reducing agents such as waste materials as input to come to capture CO₂, possibly in combination with organic carbon, into microbial biomass and products which have value.
10. The systems as described above operating under extreme conditions of pH, temperature, light, salinity, redox values, absence of mineral nitrogen.

A key feature could become to produce sulfate reducing microorganisms coated with sulfide oxidizing bacteria (dark or phototrophs) and the latter combi are catalysts in the nutrition (gut, rumen ?); in waste conversion, and so on.

In what follows, the invention is described using non-exhaustive examples illustrating the invention, which are not intended or intended to be interpreted to limit the scope of the invention.

### EXAMPLES

### Example 1:

SRBs or a consortium containing SRBs, such as *Desulfovibrio sp., Desulfotomaculum orientis, Desulfobacterium autotrophicum, Desulfobulbus sp., Desulfotomaculum sp., Desulfosporomusa sp., Desulfosporosinus sp.* and/or *Thermodesulfovibrio sp.* are brought in a trickling bed reactor in which waste water is percolating over a carrier (e.g. 200 m² surface/m³ carrier volume) at a rate near the flooding rate of the carrier and where the energy dense gas (H₂, CO, CO₂) is circulated in counter current at a rate similar to the flow rate of the liquid. The temperature range of the reactor is from 10-60 °C, but preferably around 30 °C. The reactor is operated at atmospheric pressure and consumes normal amounts of mixing energy (100-500 W installed per m³ reactor).

The latter configuration assures an intensive biomass to gas exposure and provides also for an adequate biomass retention on the carrier material. Thus volumetric loading rates of 10 g COD-H₂ per L reactor per day are attained when hydrogen is the electron donor and sulfate, the sulfur compound, is the electron acceptor. Typical conditions are a pH in the range of about 4 to about 8,5 and the hydraulic residence time is about 0,5 to about 2 days. The pH is controlled by dosing acid such as diluted H₂SO₄. The amount of H₂S in the gas phase can be controlled by operating the pH in het alkaline range. Moreover, by installing a contact interphase between the gas and a liquid which contains a reagent reacting with H₂S, as for instance a ferric or a ferro solution, an organic amine binding the H₂S, or a basic solution trapping the H₂S, the level of the gaseous H₂S can be controlled and the H₂S can be harvested as a valuable recovery product.

Exclusion of non-desired species in the sulfur reducing microorganism reactor system, for instance methanogens, can be achieved both by operating at slightly acidic conditions at the onset of the enrichment and subsequently by operating at the levels of H₂S in the liquid near the tolerance level of the sulfur reducing microorganisms i.e. about 10 mM.

For the aerobic conversion of the reduced products, a CSTR reactor is used in which the microorganisms, in particular SOBs or a consortium comprising SOBs such as as *Thiobacillus sp., Thiothrix sp., Halothiobacillus sp., Acidithiobacillus sp., Thioalkalivibrio sp., Thiomicrospira sp., Thermothrix sp., Chlorobaculum sp.* and/or *Beggiatoa sp*..are given sufficient oxygen (DO levels 0,1-10 mg/L) and are selected for rapid growth and high cellular productivity by imposing short cell residence times of the order of 0,5-4 days, typically about 2 days.
Yet, other types of reactors such as for instance trickling bed, moving bed bioreactors, membrane reactors can be used. Also the use of other drivers of the upgrading of the products of the SRB can be used such as oxidized nitrogen species (nitrite/nitrate) or light. Reactor systems work at atmospheric pressure with normal energy input levels (100-500 W installed per m³ reactor).

### Example 2: two-reactor system

Two reactor system refers to a trickle bed filter for SRBs or a consortium comprising SRBs such as *Desulfovibrio sp., Desulfotomaculum orientis, Desulfobacterium autotrophicum, Desulfobulbus sp., Desulfotomaculum sp., Desulfosporomusa sp., Desulfosporosinus sp. and*/*or Thermodesulfovibrio sp.* followed by aerobic upgrading of energy rich gases in a continuous stirred tank reactor using SOBs or a consortium comprising SOBs such as *Thiobacillus sp., Thiothrix sp., Halothiobacillus sp., Acidithiobacillus sp., Thioalkalivibrio sp., Thiomicrospira sp., Thermothrix sp., Chlorobaculum sp.* and/or *Beggiatoa sp..*

An energy rich gas, hydrogen in particular, is dosed under normal atmospheric pressure at a volumetric loading rate of the order of 10 g chemical oxygen demand per liter contact reactor per day at normal 30 °C. The gas is converted to an energy dense compound (sulfur compound, resp. organic matter compound) present in the liquid reactor phase with an efficiency in the range of 95 % or more. The principal product is sulfide, but organic acids and waxes can also be detected at levels of g per L fermentation broth.

Subsequently, the densified form of the initial energy rich gas is aerobically fermented in a continuous stirred tank reactor thereby fixing considerable amounts of CO₂, to produce microbial biomass, also called single cell protein, and possibly also valuable metabolites. The products are recovered and represent in chemical oxygen demand equivalents up to the value of about 17 %, but possibly higher in cases of advantageous co-culturing, of the original COD entering in a gaseous form (= 83 % on the initial input of the 2 step system x 0,16 (yield coefficient expressed in dry matter) x 1,3 (conversion of dry matter to COD equivalent). The biomass is rich in valuable amino acids and contains other valuable compounds such as but not limited to polyhydroxyalkanoates, polyphosphates and various forms of extracellular polymeric substances such as sulphated polysaccharides. The residual liquid of the SOB process can be recycled to the first reactor.

### Example 3: one-reactor system

The sulfur compound (thio(sulfate)) reducing reaction and the subsequent use of the products of the SRB (H₂S, possibly organic acids and/or waxes) as in Examples 1 or 2 are performed in the same reactor system, either separated by reactor conditions or by diffusion gradients. For the latter, both reactions can occur in microbial granular systems or in reactors equipped with membrane systems to keep them separated.

### REFERENCES

Adedayo, M. R., Ajiboye, E. A., Akintunde, J. K., & Odaibo, A. (2011). Single Cell Proteins: As Nutritional Enhancer. Advances in Applied Science Research, 2(5), 396-406. Retrieved from http://pelagiaresearchlibrary.com/advances-in-applied-science/vol2-iss5/AASR-2011-2-5-396-409.pdf
Anupama, & Ravindra, P. (2000). Value-added food: Single cell protein. Biotechnology Advances, 18(6), 459-479. https://doi.org/10.1016/S0734-9750(00)00045-8
Badziong, W., & Thauer, R. K. (1978). Growth yields and growth rates of Desulfovibrio vulgaris (Marburg) growing on hydrogen plus sulfate and hydrogen plus thiosulfate as the sole energy sources. Archives of Microbiology, 117(2), 209-214. https://doi.org/10.1007/BF00402310
Badziong, W., Thauer, R. K., & Zeikus, J. G. (1978). Isolation and characterization of Desulfovibrio growing on hydrogen plus sulfate as the sole energy source. Archives of Microbiology, 116(1), 41-49. https://doi.org/10.1007/BF00408732
Bharathi, P. A. L. (2008). Sulfur Cycle. In E. B.V. (Ed.), Global Ecology (pp. 3424-3431). Panaji, India: National Institute of Oceanography. https://doi.org/10.1007/978-3-642-27833-4_1539-2
Brandis, A., & Thauer, R. K. (1981). Growth of Desulfovibrio species on Hydrogen and Sulphate as Sole Energy Source. Microbiology, 126(1), 249-252. https://doi.org/10.1099/00221287-126-1-249
Brysch, K., Schneider, C., Fuchs, G., & Widdel, F. (1987). Lithoautotrophic growth of sulfate-reducing bacteria, and description of Desulfobacterium autotrophicum gen. nov., sp. nov. Arch Microbiol (1987), 148, 264-274. https://doi.org/10.1016/S0168
Caffrey, S. M., & Voordouw, G. (2010). Effect of sulfide on growth physiology and gene expression of Desulfovibrio vulgaris Hildenborough. Antonie van Leeuwenhoek, International Journal of General and Molecular Microbiology, 97(1), 11-20. https://doi.org/10.1007/s10482-009-9383-y
Colleran, E., Finnegan, S., & Lens, P. (1995). Anaerobic treatment of sulphate-containing waste streams. Antonie van Leeuwenhoek, 67(1), 29-46. https://doi.org/10.1007/BF00872194
Cypionka, H., & Pfennig, N. (1986). Growth yields of Desulfotomaculum orientis with hydrogen in chemostat culture. Archives of Microbiology, 396-399.
Ebrahimi, S., Kleerebezem, R., van Loosdrecht, M. C. M., & Heijnen, J. J. (2003). Kinetics of the reactive absorption of hydrogen sulfide into aqueous ferric sulfate solutions. Chemical Engineering Science, 58(2), 417-427. https://doi.org/10.1016/S0009-2509(02)00522-5
EPA. (n.d.). EPA Design Odor and Corrosion Control in Sanitary Systems and Treament Plants.
Gholami, Z., Angaji, M. T., Gholami, F., & Alavi, S. A. R. (2009). Reactive Absorption of Hydrogen Sulfide in Aqueous Ferric Sulfate Solution. World Academy of Science, Engineering and Technology, 49 (January 2009), 208-210.
Hao, O. J. (2003). Sulphate-reducing bacteria. In The Handbook of Water and Wastewater Microbiology (pp. 459-469). University of Maryland, MD 20742, USA: Department of Civil Engineering.
Hedrich, S., & Johnson, D. B. (2013). Aerobic and anaerobic oxidation of hydrogen by acidophilic bacteria. FEMS Microbiology Letters, 349(1), 40-45. https://doi.org/10.1111/1574-6968.12290
Klatt, J. M., & Polerecky, L. (2015). Assessment of the stoichiometry and efficiency of CO2 fixation coupled to reduced sulfur oxidation. Frontiers in Microbiology, 6(MAY). https://doi.org/10.3389/fmicb.2015.00484
Lens, P. N. L., & Kuenen, J. G. (2001). The biological sulfur cycle: Novel opportunities for environmental biotechnology. Water Science and Technology, 44(8), 57-66.
Liamleam, W., & Annachhatre, A. P. (2007). Electron donors for biological sulfate reduction. Biotechnology Advances, 25(5), 452-463. https://doi.org/10.1016/j.biotechadv.2007.05.002
Lovley, D. R., Dwyer, D. F., & Klug, M. J. (1982). Kinetic Analysis of Competition Between Sulfate Reducers and Methanogens for Hydrogen in Sediments. Applied and Environmental Microbiology, 43(6), 1373-1379.
Lupton, F. S., & Zeikus, J. G. (1984). Physiological basis for sulfate-dependent hydrogen competition between sulfidogens and methanogens. Current Microbiology, 11(1), 7-12. https://doi.org/10.1007/BF01567568
Mesa, M. M., Andrades, J. A., Macías, M., & Cantero, D. (2004). Biological oxidation of ferrous iron: Study of bioreactor efficiency. Journal of Chemical Technology and Biotechnology, 79(2), 163-170. https://doi.org/10.1002/jctb.956
Nasseri, A. T., Rasoul-Amini, S., Morowvat, M. H., & Ghasemi, Y. (2011). Single cell protein: Production and process. American Journal of Food Technology. https://doi.org/10.3923/ajft.2011.103.116
Okabe, S., Nielsen, P. H., & Characklis, W. G. (1992). Factors affecting microbial sulfate reduction by Desulfovibrio desulfuricans in continuous culture: Limiting nutrients and sulfide concentration. Biotechnology and Bioengineering, 40(6), 725-734. https://doi.org/10.1002/bit.260400612
Prado-Rubio, O. A., Jørgensen, J. B., & Jørgensen, S. B. (2010). Systematic model analysis for single cell protein (SCP) production in a U-loop reactor. Computer Aided Chemical Engineering (Vol. 28). Elsevier B.V. https://doi.org/10.1016/S1570-7946(10)28054-9
Reis, M. A. M., Almeida, J. S., Lemos, P. C., & Carrondo, M. J. T. (1992). Effect of hydrogen sulfide on growth of sulfate reducing bacteria. Biotechnology and Bioengineering, 40(5), 593-600. https://doi.org/10.1002/bit.260400506
Ritala, A., Häkkinen, S. T., Toivari, M., & Wiebe, M. G. (2017). Single cell protein-state-of-the-art, industrial landscape and patents 2001-2016. Frontiers in Microbiology, 8(OCT). https://doi.org/10.3389/fmicb.2017.02009
Robinson, J. A., & Tiedje, J. M. (1984). Competition between sulfate-reducing and methanogenic bacteria for H2 under resting and growing conditions. Archives of Microbiology, 137(1), 26-32. https://doi.org/10.1007/BF00425803
Schwartz, E., Fritsch, J., & Friedrich, B. (2013). H2-Metabolizing Prokaryotes. In E. Rosenberg et al. (Ed.), The Prokaryotes - Prokaryotic Physiology and Biochemistry (pp. 120-174). Berlin, Germany: Springer. https://doi.org/10.1007/978-3-642-30141-4_65
Suman, G., Nupur, M., Anuradha, S., & Pradeep, B. (2015). Single Cell Protein Production: A Review. International Journal of Current Microbiology and Applied Sciences, 4(9), 251-262. https://doi.org/10.1016/S0167-8760(01)00179-9
Ugbogu, E. A., & Ugbogu, O. C. (2016). A Review Of Microbial Protein Production: Prospects And Challenges. FUW Trends in Science & Technology Journal, 1(July), 182-185.
Van Houten, R. T., Van Der Spoel, H., Van Aelst, A. C., Hulshoff Pol, L. W., & Lettinga, G. (1996). Biological sulfate reduction using synthesis gas as energy and carbon source. Biotechnology and Bioengineering, 50(2), 136-144. https://doi.org/ 10.1002/(SICI)1097-0290(19960420)50:2<136::AID-BIT3>3.0.CO;2-N
Visser, A., Beeksma, I., van der Zee, F., Stams, A. J. M., & Lettinga, G. (1993). Anaerobic degradation of volatile fatty acids at different sulphate concentrations. Appl Microbiol Biotechnol, 40, 549-556.
Yu, J., Dow, A., & Pingali, S. (2013). The energy efficiency of carbon dioxide fixation by a hydrogen-oxidizing bacterium. International Journal of Hydrogen Energy, 38(21), 8683-8690. https://doi.org/10.1016/j.ijhydene.2013.04.153
Shivvers, D & Brock, T (1973). Oxidation of elemental sulfur by Sulfolobu acidocalarius. J Bacteriol 114: 706-710.
Sublette, K. (1988). Production of microbial biomass protein from atutophic fermentation of hydrogen sulfide. Biotech Bioeng 32:408-409
J.M. Klatt and L. Polerecky 2015 Assessment of the stoichiometry and efficiency of CO2 fixation coupled to reduced sulfur oxidation. Frontiers Microbiology (MayKnikerbocker, C, Nordstrom, D.K & Southam, G. 2000.The role of blebbing in overcoming the hydrophobic barrier during biooxidagtion of elemental sulfur by Thiobacillus thiooxidans. Chemical Geology 169: 425-439.
Zhang, C, Xia, J,Ouyang, X, Nie, Z & Qiu, G. 2009. Cellular acclimation of Acidithiobacillus ferrooxidans to sulfur metabolism. Minerals and Metallurgical Processes 26: 30-33
Qian, Z, Tianwei,H, Mackey, H, van Loosdrecht, M.2019. Recent advances in dissimilatory sulfate reduction: from metabolic study to application. Water Research 150:162-181
Volkov,I, Rozanov, A. 1983. The sulfur cycle in the oceans. The global biogeochemical sulfur cycle.SCOPE 19: 357-423
Suylen, G, Stefen, G & keunen, J.1986. Chemolithotrophic potential of a Hyphomicrobium species capable of growth on methylated Sulphur compounds. Archiv Micrbobiol 146: 192-198
Wahlen, B, Oswald,W, Seefeldt L, Lance, C & Barney, B. 2009. Purification, characterization, and potential bacterial wax
Production rle of an NADPH-dependent fatty aldehyde reductase from Marinobacter aquaeolei VT8. Applied and Environmental Microbiology 75: 2758-2765
Adam, N & Perner, M. 2018. Microbially mediated hydrogen cycling in deep-see hydrothermal vents. Frontiers in Microbiology 9: 1-32
Jansen a, Ruitenberg A & Buisman C 2001. Industrial applications of new sulphur biotechnology. Water Science and Technology 44:85-90
Saer, R & Blankenship,R 2017. Light harvesting in phototrophic bacteria structure and function. Biochemical Journal 474: 2107-2131
Garcia, G, Diniz, R,Bicalho, S, Franco, V, Pereira, A, Brandt,E, Etchebehere, C, Chernicharo, C & de Araujo, J. Micobial community and Sulphur behavior in phototrophic reactors treating UASB effluent under different operational conditions.2017 Int Biodeterioration & Biodegration. 119: 486-498
Ysain,M, Jeong,Y, Park, S, Jeong J, Lee, E, Lowitt, R, Kim, B, Lee, J, Chang, I 2015. Microbial synthesis gas utilization and ways to resolve kinetic and mass transfer limitations. Bioresource Technology 177: 361-374
Kooli, W, Junier, T, Shakya, M, Monachon, M, Davenport, K, Valdeeswaran, K, Vernudachi, A, Marozau, I, Monrouzeau, T, Gleasner, C, MicMurry,K, Lienhard, R, Rufener,L, Perret, J, Sereda, O, Chain, P, Joseph E. & Junier, P.2019 Rmedial treatment of corroded iron objects by environmental Aeromonas isolates. Appl Env Microbil 85:1-17
Patel, K, Singh R, Kumar,A, Jeong, J, Jeong,S, Kalia, V, Kim I and Lee, J 2019. Biological methanol production by immobilized Methylocella tundrae using simulate biohythane as a feed. Bioresource Technology: in press
Molitor, B, Mishra, A and Angenent, L. 2019. Power-to-protein: converting renewable electric power and carbon dioxide into single cell protein with a two-stage bioprocess. Energy an Environmental Science: in press
Gang, Y, Ebrahim, A, Feist, A.M, Embree, M, Zhang,T, Lovely,D and Zengler, K. 2012. Sulfide-driven microbial electrosynthesis. Env Sci and Technology 47:568-573
Nelson, D.C., Jorgensen, B B and Revsbech, N.P. 1986. Growth pattern and yield of a chemoautotrophic Beggiatoa sp. in oxygen-sulfide microgradients. Applied and Environmental Microbiology 52:225-233
Jin Qian, et al., Thiosulfate as the electron acceptor in sulfer bioconversion associated process SBAP for sewage treatment Water research 163 2019.

## Claims

1. Method for producing biomass or derivatives thereof, comprising converting a sulfur compound to hydrogen sulfide by sulfate reducing microorganisms and subsequently converting said hydrogen sulfide into biomass by means of sulfide oxidizing bacteria (SOB), said conversions are mediated by electron transfer of one or more energy rich gases.

2. Method according to claim 1, wherein said energy rich gases are selected from hydrogen, CO, H₂S, PH₃, CH₄, volatile organics, gases produced by fermentation, and mixes of such gases.

3. Method according to claims 1 to 2, wherein said energy rich gas is hydrogen.

4. Method according to any of the previous claims, wherein said biomass derivatives are chosen from proteins, single-cell protein, polyhydroxy butyrate and analogues, polyphosphates, carbohydrates, cell wall components preferably exopolysaccharides preferably sulfated polysaccharides, lipids, extracellular dissolved organic carbon compounds and metabolites.

5. Method according to any of the previous claims, wherein said sulfate reducing microorganisms are selected from the sulfate-reducing bacteria (SRB) and sulfate-reducing archaea (SRA).

6. Method according to any of the previous claims, wherein said converting of sulfate to hydrogen sulfide occurs under anaerobic circumstances.

7. Method according to any of the previous claims, wherein the reducing of said sulfur compound occurs in the presence of a carbon dioxide source.

8. Method according to any of the previous claims, wherein said sulfur compound is sulfate or thiosulfate.

9. Method according to any of the previous claims, wherein said converting of hydrogen sulfide into biomass or derivatives occurs under aerobic conditions.

10. Method according to any of the previous claims, wherein said conversion reactions occur in a bioreactor.

11. Method according to claim 10, wherein said conversion reactions occur in separate compartments of a bioreactor, or separate bioreactors.

12. Method according to any of the previous claims 1 to 9, wherein the hydrogen sulfide conversion reaction occurs in open systems such as soils, composts, activated sludges, and other biotic or abiotic fermentation systems.

13. Method according to any of the previous claims, wherein waste material is used as starting material for the conversion reactions, preferably biowaste.

14. Method for producing biomass or derivatives thereof, from material comprising one or more sulfur compounds, said method comprises the following steps:
- converting said sulfur compound(s) to hydrogen sulfide by sulfate reducing microorganisms under anaerobic conditions; and subsequently
- converting obtained hydrogen sulfide by into biomass by means of sulfide oxidizing bacteria under aerobic conditions;
wherein said conversions are mediated by energy rich gases, and said conversions take place in one or more bioreactors.

15. A bioreactor system for producing biomass or derivatives thereof, using sulfate reducing microorganisms and sulfide oxidizing bacteria, the system comprising:
- a first compartment comprising at least one sulfate reducing microorganism culture, one gas inlet configured to supply an amount of energy rich gas, preferably at least one gas outlet, at least one fluid inlet, and at least one fluid outlet;
- a second compartment comprising at least one sulfide oxidizing bacteria culture.
